**Europäisches Patentamt**

**European Patent Office**

**Office européen des brevets**

(19)

(11) Veröffentlichungsnummer: **0 204 737 B1**

(12) **EUROPÄISCHE PATENTSCHRIFT**

(45) Veröffentlichungstag der Patentschrift: **08.04.92**

(51) Int. Cl.⁵: **A61K 47/12**

(21) Anmeldenummer: **85905757.2**

(22) Anmeldetag: **06.12.85**

(86) Internationale Anmeldenummer:
**PCT/CH85/00172**

(87) Internationale Veröffentlichungsnummer:
**WO 86/03413 (19.06.86 86/13)**

Die Akte enthält technische Angaben, die nach dem Eingang der Anmeldung eingereicht wurden und die nicht in dieser Patentschrift enthalten sind.

(54) **PRÄPARAT ZUR INTRAMUSKULÄREN INJEKTION VON MEDIKAMENTEN, VITAMINEN ODER IMPFSTOFFEN.**

(30) Priorität: **07.12.84 CH 5860/84**

(43) Veröffentlichungstag der Anmeldung:
**17.12.86 Patentblatt 86/51**

(45) Bekanntmachung des Hinweises auf die Patenterteilung:
**08.04.92 Patentblatt 92/15**

(84) Benannte Vertragsstaaten:
**AT BE CH DE FR GB IT LI LU NL SE**

(56) Entgegenhaltungen:
**DE-B- 1 007 022**
**DE-B- 1 062 389**
**FR-A- 1 023 821**
**US-A- 4 081 527**

**CHEMICAL ABSTRACTS, vol. 89, no. 4, 24 Juli 1978, Columbus, OH (US); p. 407, no. 30788t**

**CHEMICAL ABSTRACTS, vol. 100, no. 26, Juni 1984, Columbus, OH (US); p. 346, no. 215543y**

**RÖMPP's CHEMIE LEXIKON, Band 1, 1979; Seite 573**

**ROTE LISTE, 1981, Edition Cantor; Stichwort "Macalvit R -K"**

(73) Patentinhaber: **Hadziselimovic, Faruk, Prof. Dr. Fichtenwaldstrasse 22 CH-4142 Münchenstein(CH)**

(72) Erfinder: **Hadziselimovic, Faruk, Prof. Dr. Fichtenwaldstrasse 22 CH-4142 Münchenstein(CH)**

(74) Vertreter: **Braun jr., André Patentanwalt VSP Murtengasse 5 CH-4051 Basel(CH)**

**Beschreibung**

Die Erfindung betrifft die Verwendung von Ca-glucoheptonat in racemischer, D- oder L-Form, gemäss Patentanspruch 1.

Intramuskuläre Injektionen von Medikamenten oder Vitaminen und Impfungen können sehr schmerzhaft sein. Um diese Reaktionen zu verringern oder zu vermeiden, wurden bisher als pharmazeutische Zusatzstoffe Lokalanästetika, wie z.B. Lidocainhydrochlorid oder Mepivacainhydrochlorid, dem Medikament oder Vitamin oder dem Impfstoff zugesetzt.

Nun ist es z.B. allerdings bekannt, dass Lokalanästhetika und auch viele Medikamente, insbesondere Penicilline, bei ihrer Injektion einen anaphylaktischen Schock auslösen können. Diese Reaktion gilt z.B. in den USA als die am häufigsten auftretende Todesursache in medizinischen Privatpraxen, und in Frankreich sterben 2 auf 10'000 Falle pro Jahr daran.

Zweck der Erfindung war es deshalb, einen Zusatzstoff zu finden, der zwar schmerzlose Injektionen von Medikamenten, Vitaminen und Impfstoffen erlaubt, jedoch nicht die gefährlichen Nebenwirkungen bekannter lokaler Schmerzmittel aufweist.

Es wurde nun überraschenderweise gefunden, dass durch Zugabe von Ca-glucoheptonat zum Medikament, Vitamin oder zum Impfstoff der bei intramuskulärer Injektion auftretende Schmerz stark gemindert bzw. gänzlich vermieden werden kann, die gefährlichen Allergiereaktionen von Lokalanästhetika vermieden und überdies die medikamentös bedingten Allergiereaktionen verhindert oder stark gemindert werden können.

Das erfindungsgemässe Präparat wird durch Mischen des Medikamentes, Vitamins oder Impfstoffes mit Ca-glucoheptonat in an sich bekannter Weise hergestellt. Das Ca-glucoheptonat wird dabei normalerweise in gelöster Form z.B. dem Medikament zugesetzt. Als Lösungsmittel kann man $H_2O$ destilliert oder bidestilliert, physiologische NaCl-Lösung oder 5 % Glucoselösung verwenden. Diesem wird etwa 0,01 % bis 25 %, insbesondere ca. 10 %, Ca-glucoheptonat beigemengt und darin aufgelöst. Das solchermassen gelöste Ca-glucoheptonat kann dann mit dem Medikament, Vitamin oder Impfstoff vermischt werden, wobei die Konzentration des Ca-glucoheptonats im Lösungsmittel und die Zugabe-Menge zum Medikament, Vitamin oder Impfstoff von der Art und Menge des zu injizierenden Stoffes abhängig ist und beispielsweise bei Penicillin G pro 300'000 I.E. 0,5 bis 2,5 ml 10 % Ca-glucoheptonatlösung betragen kann.

Es besteht auch die Möglichkeit, Ca-glucoheptonat durch chemische Reaktion an das Medikament, Vitamin oder den Impfstoff zu koppeln.

Grundsätzlich wird durch Erhöhung der Konzentration und/oder der Menge an gelöstem Ca-glucoheptonat die schmerzlindernde Wirkung bei Injektionen erhöht.

Intramuskuläre Injektionen von Depotantibiotika und Vitamin B eignen sich dabei besonders gut für die Verwendung von Ca-glucoheptonat zen zum Zwecke schmerzloser Injektion.

Die Erfindung wird durch die folgenden Beispiele weiter erläutert.

Beispiel 1

In eine desinfizierte Trockenampulle von Benzathin-benzylpenicillin 600'000 I.E. wird 2 ml einer 10 %-igen wässrigen Ca-glucoheptonatlösung injiziert und solange geschüttelt, bis deren Inhalt suspendiert ist. Die Suspension wird einer Versuchsperson intraglutäal, loco classico, injiziert. Die Versuchsperson empfindet dabei nicht die typischen Schmerzen, die üblicherweise durch intramuskuläre Penicillininjektionen entstehen.

Beispiel 2

1 ml Vitamin $B_1$ (= 100 $\mu$g Thiamin) wird in 3 ml einer 10 %-igen wässrigen Ca-glucoheptonatlösung suspendiert und dann der Versuchsperson analog Beispiel 1 injiziert. Die Versuchsperson empfindet dabei nicht die typischen Schmerzen, die üblicherweise durch intramuskuläre Vitamin-$B_1$-Injektionen entstehen.

Beispiel 3

1 ml Vitamin $B_1$ (= 100 $\mu$g Thiamin) wird in 1,5 ml einer 10 %-igen wässrigen Ca-glucoheptonatlösung suspendiert und dann der Versuchsperson analog Beispiel 1 injiziert. Die Versuchsperson empfindet dabei nicht die typischen Schmerzen, die üblicherweise durch intramuskuläre Vitamin-$B_1$-Injektionen entstehen.

Beispiel 4

In eine desinfizierte Trockenampulle von Benzylpenicillin-Na 500'000 I.E. werden 2 ml einer 5 %-igen wässrigen Ca-glucoheptonatlösung injiziert und solange geschüttelt, bis deren Inhalt suspendiert ist. Die Suspension wird einer Versuchsperson intraglutäal, loco classico, injiziert. Die Versuchsperson empfindet dabei nicht die typischen Schmerzen, die üblicherweise durch intramuskuläre Penicillininjektionen entstehen.

Beispiel 5

Mumpsvax® (Mumps-Lebendvirus-Vakzine, MSD) mit mindestens 5'000 $TCID_{50}$/Dosis wird in 0,5 ml 10 %-iger Ca-glucoheptonatlösung suspendiert und einer Versuchsperson subkutan injiziert. Die Versuchsperson empfindet dabei keine typischen Schmerzen.

Beispiel 6

M-M-$R_{II}$® (Masern-, Mumps- und Röteln-Lebendvirus-Vakzine, MSD), zusammengestzt aus 1. ATTENUVAX® (Masern-Lebendvirus-Vakzine, MSD), 2. MUMPSVAX® (Mumps-Lebendvirus-Vakzine, MSD) und 3. MERUVAX® (Röteln-Lebendvirus-Vakzine, MSD) und enthaltend mindestens 1'000 $TCID_{50}$ (Gewebekultur-Infektionsdosen) Masern-Lebendvirus-Vakzine, 5'000 $TCID_{50}$ Mumps-Lebendvirus-Vakzine und 1'000 $TCID_{50}$ Röteln-Lebendvirus-Vakzine, werden in 1 ml 10 %-iger Ca-glucoheptonatlösung suspendiert und einer Versuchsperson subkutan injiziert. Die Applikation ist praktisch schmerzfrei.

**Patentansprüche**
**Patentansprüche für folgende Vertragsstaaten : BE, CH, DE, FR, GB, IT, LI, LU, NL, SE**

1. Verwendung von Ca-glucoheptonat in racemischer, D- oder L-Form, zur Herstellung eines in Kombination mit einem Medikament, Vitamin oder Impfstoff intramuskulär schmerzlos injizierbaren Präparates.

2. Verwendung von Ca-glucoheptonat nach Patentanspruch 1, dadurch gekennzeichnet, dass das Ca-glucoheptonat in $H_2O$ destilliert oder bidestilliert, physiologischer NACl-Lösung oder 5% Glucoselösung gelöst dem Medikament, Vitamin oder Impfstoff zugesetzt wird.

3. Verwendung von Ca-glucoheptonat nach einem der Patentansprüchen 1 oder 2, dadurch gekennzeichnet, dass Ca-glucoheptonat in einer Konzentration im Lösungsmittel von 0,01 bis 25%, insbesondere von 10%, verwendet wird.

4. Verwendung von Ca-glucoheptonat nach einem der Patentansprüche 1-3, dadurch gekennzeichnet, dass das Medikament Penicillin ist.

5. Verwendung von Ca-glucoheptonat nach Patentanspruch 4, dadurch gekennzeichnet, dass pro 300'000 I.E. Penicillin 0,5 bis 2,5 ml, insbesondere 1 bis 1,5 ml, 10% Ca-glucoheptonat, zugesetzt wird.

6. Verwendung von Ca-glucoheptonat nach einem der Patentansprüche 1-3, dadurch gekennzeichnet, dass das Vitamin Vitamin B ist.

7. Verwendung von Ca-glucoheptonat nach einem der Patentansprüche 1-3, dadurch gekennzeichnet, dass der Impfstoff eine Mumps-Lebendvirus-Vakzine, MSD, wie Mumpsvax®, oder eine Masern-, Mumps- und Röteln-Lebendvirus-Vakzine, MSD, wie M-M-$R_{II}$®, ist.

**Patentansprüche für folgenden Vertragsstaat : AT**

1. Verfahren zum Herstellen eines ein Medikament, Vitamin oder Impfstoff enthaltenden, intramuskulär schmerzlos injizierbaren Präparates, dadurch gekennzeichnet, dass man Ca-glucoheptonat in racemischer, D- oder L-Form mit dem Medikament, Vitamin oder Impfstoff mischt.

2. Verfahren nach Anspruch 1, dadurch gekennzeichnet, dass man das Ca-glucoheptonat gelöst in destilliertem oder bidestilliertem Wasser, in physiologischer Kochsalzlösung oder in 5%iger Glukoselösung mit dem Medikament, Vitamin oder Impfstoff mischt.

3. Verfahren nach Anspruch 2, dadurch gekennzeichnet, dass man mit dem Medikament, Vitamin oder Impfstoff eine Lösung von Ca-glucoheptonat mischt, in der CA-glucoheptonat, bezogen auf das Lösungsmittel in einer Konzentration von 0,01 bis 25%, insbesondere von 10% enthalten ist.

4. Verfahren nach einem der Ansprüche 1 bis 3, dadurch gekennzeichnet, dass man Ca-glucoheptonat mit Penicillin mischt.

5. Verfahren nach Anspruch 4, dadurch gekennzeichnet, dass man je 300 000 I.E. Penicillin mit 0,5 bis 2,5 ml, insbesodnere 1 bis 1,5 ml einer 10%igen Ca-glucoheptonat-Lösung mischt.

6. Verfahren nach einem der Ansprüche 1 bis 3, dadurch gekennzeichnet, dass man Ca-glucoheptonat mit Vitamin B mischt.

7. Verfahren nach einem der Ansprüche 1 bis 3, dadurch gekennzeichnet, dass man Ca-glucopheptonat mit einer Mumps-Lebendvirus-Vakzine, MSD, wie Mumpsvax®, oder einer Masern-, Mumps- und Röteln-Lebendvirus-Vakzine, MSD, wie M-M-$R_{II}$®, mischt.

**Claims**
**Claims for the following Contracting State : BE, CH, DE, FR, GB, IT, LI, LU, NL, SE**

1. Use of Ca-glucoheptonate in racemic, D- or L-form for producing a preparation which can be painlessly injected by intramuscular route in conjunction with a drug, vitamin or vaccine.

2. Use of Ca-glucoheptonate according to claim 1, characterised in that the Ca-glucoheptonate is added to the drug, vitamin or vaccine after being dissolved in distilled or double-distilled $H_2O$, physiological saline solution or 5% glucose solution.

3. Use of Ca-glucoheptonate according to claim 1 or 2, characterised in that Ca-glucoheptonate is used in a concentration in the solvent of from 0.01 to 25%, more particularly 10%.

4. Use of Ca-glucoheptonate according to one of claims 1 to 3, characterised in that the drug is penicillin.

5. Use of Ca-glucoheptonate according to claim 4, characterised in that 0.5-2.5 ml, more particularly 1-1.5 ml, of 10% Ca-glucoheptonate are added per 300,000 I.U. of penicillin.

6. Use of Ca-glucoheptonate according to one of claims 1 to 3, characterised in that the vitamin is vitamin B.

7. Use of Ca-glucoheptonate according to one of claims 1 to 3, characterised in that the vaccine is a mumps live virus vaccine, MSD, such as Mumpsvax®, or a measles, mumps and rubella live virus vaccine, MSD, such as M-M-R$_{II}$®.

**Claims for the following Contracting State : AT**

1. Process for preparing a preparation which can be injected painlessly by intramuscular route and contains a drug, vitamin or vaccine, characterised in that Ca-glucoheptonate in the racemic, D- or L-form is mixed with the drug, vitamin or vaccine.

2. Process according to claim 1, characterised in that the Ca-glucoheptonate, dissolved in distilled or double-distilled water, in physiological saline solution or in 5% glucose solution, is mixed with the drug, vitamin or vaccine.

3. Process according to claim 2, characterised in that the drug, vitamin or vaccine is mixed with a solution of Ca-glucoheptonate in which Ca-glucoheptonate is contained in a concentration of from 0.01 to 25%, more particularly 10%, based on the solvent.

4. Process according to one of claims 1 to 3, characterised in that Ca-glucoheptonate is mixed with penicillin.

5. Process according to claim 4, characterised in that 300,000 I.U. of penicillin are mixed with 0.5-2.5 ml, more particularly 1-1.5 ml, of a 10% Ca-glucoheptonate solution.

6. Process according to one of claims 1 to 3, characterised in that Ca-glucoheptonate is mixed with vitamin B.

7. Process according to one of claims 1 to 3, characterised in that Ca-glucoheptonate is mixed with a mumps live virus vaccine, MSD, such as Mumpsvax®, or a measles, mumps and rubella live virus vaccine, MSD, such as M-M-R$_{II}$®.

**Revendications**
**Revendications pour les Etats contractants suivants : BE, CH, DE, FR, GB, IT, LI, LU, NL, SE**

1. Utilisation du glucoheptonate de calcium, sous forme racémique, sous forme D ou sous forme L, pour la fabrication d'une préparation injectable par voie intramusculaire et sans douleur, en combinaison avec un médicament, une vitamine ou un vaccin.

2. Utilisation du glucoheptonate de calcium selon la revendication 1, caractérisée en ce que on l'ajoute au médicament, à la vitamine ou au vaccin, le glucoheptonate de calcium dissous dans de l'eau distillée ou bidistillée, dans une solution physiologique de NaCl ou dans une solution de glucose à 5 %.

3. Utilisation du glucoheptonate de calcium selon l'une des revendications 1 et 2, caractérisée en ce que le glucoheptonate du calcium est utilisé en une concentration de 0,01 à 25 %, en particulier de 10 %, dans le solvant.

4. Utilisation du glucoheptonate de calcium selon l'une des revendications 1 à 3, caractérisée en ce que le médicament est de la pénicilline.

5. Utilisation du glucoheptonate de calcium selon la revendication 4, caractérisée en ce que l'on ajoute, pour 300 000 U.I. de pénicilline, de 0,5 à 2,5 ml, et en particulier de 1 à 1,5 ml, d'une solution de glucoheptonate de calcium à 10 %.

6. Utilisation du glucoheptonate de calcium selon l'une des revendications 1 à 3, caractérisée en ce que la vitamine est de la vitamine B.

**7.** Utilisation du glucoheptonate de calcium selon l'une des revendications 1 à 3, caractérisée en ce que le vaccin est un vaccin contre les oreillons à base de virus vivants, MSD, comme Mumpsvax®, ou un vaccin contre les oreillons, la rougeole et la rubéole, à base de virus vivants, MSD, comme M-M-R$_{II}$®.

**Revendications pour les Etat contractant suivant: AT**

**1.** Procédé de fabrication d'une préparation injectable par voie intramusculaire et sans douleur, contenant un médicament, une vitamine ou un vaccin, caractérisé en ce que l'on mélange du glucoheptonate de calcium, sous forme racémique, sous forme D ou sous forme L, au médicament, à la vitamine ou au vaccin.

**2.** Procédé selon la revendication 1, caractérisé en ce que l'on mélange, au médicament, à la vitamine ou au vaccin, le glucoheptonate de calcium dissous dans de l'eau distillée ou bidistillée, dans une solution physiologique de chlorure de sodium ou dans une solution de glucose à 5 %.

**3.** Procédé selon la revendication 2, caractérisé en ce que l'on mélange, au médicament, à la vitamine ou au vaccin, une solution de glucoheptonate de calcium qui contient le glucoheptonate de calcium en une concentration de 0,01 à 25 %, et en particulier de 10 %. par rapport au solvant.

**4.** Procédé selon l'une des revendications 1 à 3, caractérisé en ce que l'on mélange du glucoheptonate de calcium avec de la pénicilline.

**5.** Procédé selon la revendication 4, caractérisé en ce que l'on mélange, pour 300 000 U.I. de pénicilline, de 0,5 à 2,5 ml, et en particulier de 1 à 1,5 ml, d'une solution de glucoheptonate de calcium à 10%.

**6.** Procédé selon l'une des revendications 1 à 3, caractérisé en ce que l'on mélange du glucoheptonate de calcium avec de la vitamine B.

**7.** Procédé selon l'une des revendications 1 à 3, caractérisé en ce que l'on mélange du glucoheptonate de calcium avec un vaccin contre les oreillons à base de virus vivants, MSD, comme Mumpsvax®, ou avec un vaccin contre les oreillons, la rougeole et la rubéole, à base de virus vivants, MSD, comme M-M-R$_{II}$®.